# EUROPEAN PATENT SPECIFICATION

(11) **EP 4 411 384 B1**
(45) Date of publication and mention of the grant of the patent: **25.11.2026**
(21) Application number: 23214880.9
(22) Date of filing: 07.12.2023
(51) Int. Cl.: G01N 35/02, B01L 7/00, C12Q 1/6844

(54) **AMPLIFYING AND TESTING DEVICE, AND CARRIER LOADING METHOD FOR AMPLIFYING AND TESTING**
VERSTÄRKUNGS- UND TESTVORRICHTUNG UND TRÄGERLADEVERFAHREN ZUR VERSTÄRKUNG UND PRÜFUNG
DISPOSITIF D'AMPLIFICATION ET DE TEST, ET PROCÉDÉ DE CHARGEMENT DE PORTEUSE POUR AMPLIFICATION ET TEST

(30) Priority: 31.12.2022 CN 202211736930
(43) Date of publication of application: 07.08.2024
(73) Proprietor: Shenzhen New Industries Biomedical Engineering Co., Ltd., Shenzhen, Guangdong 518122 (CN)
(72) Inventor: HE, Guoyao, Shenzhen, 518122 (CN); LI, Peng, Shenzhen, 518122 (CN); WANG, Qiuming, Shenzhen, 518122 (CN); WANG, Yixian, Shenzhen, 518122 (CN); ZHENG, Xiaolin, Shenzhen, 518122 (CN); XIAO, Hao, Shenzhen, 518122 (CN)
(74) Representative: Studio Torta S.p.A.

(56) References cited:
- WO-A1-2022/226868
- CN-U- 213 475 983
- US-A1- 2008 176 290
- US-A1- 20240 042 431

## Description

### Technical Field

The present invention relates to the field of medical apparatuses, and particularly relates to an amplifying and testing device, and a carrier loading method for amplifying and testing.

### Background

An amplifying and testing device in the related art consists of a heating device and a test device. A carrier accommodating a reagent needs to be conveyed to the test device from the outside of the amplifying and testing device in order to perform the test. In the related art, the carrier accommodating the reagent is conveyed by horizontal movement of the heating device or the test device, but the heating device or the test device in the related art has a complicated structure and occupies a large area. If the heating device or the test device is used to convey the carrier accommodating the reagent in and out, the amplifying and testing device will require a large area. However, an existing environment requires a high throughput of the amplifying and testing device. In order to achieve this target, a plurality of amplifying and testing devices are required for processing in parallel, but since the area required for one amplifying and testing device is large, to accommodate the plurality of amplifying and testing devices will result in a very large area is required for an entire nucleic acid all-in-one machine, which does not take advantage of the miniaturization development.

Therefore, the large area occupied by amplifying and testing devices in the related art does not facilitate the setup of plurality of amplifying and testing devices within the nucleic acid all-in-one machine to achieve the goal of high throughput.

Examples of Sustainable Sample-adding Fluorescent PCR Instrument according to the prior art are known from CN213475983U. Examples of Apparatus for High Throughput Chemical Reactions according to the prior art are known from US2008176290A1. Examples of Chip Loading Structure, Analysis Device and Analysis System according to the prior art are known from WO2022226868A1.

### Summary

A main objective of the present invention is to provide an amplifying and testing device, and a carrier loading method for amplifying and testing, so as to solve the problem of a large area occupied by an amplifying and testing device in the related art.

The invention is set out in the appended set of claims.

In order to achieve the above objective, according to an aspect of the present invention, an amplifying and testing device is provided. The amplifying and testing device includes: a support, being provided with a test window; a test device arranged on the support and located at the test window; a reagent loading device, including a bracket and a carrier removably arranged on the bracket, the bracket is movably arranged on the support in a transverse direction, and the carrier moves along with the bracket, such that the carrier has a storage position located below the test window and a loading position moving out of the support; and a heating device movably arranged on the support in a vertical direction and capable of driving the carrier to lift, so as to lift the carrier to a test position cooperating with the test device in a butting mode.

In some embodiments, the reagent loading device further includes a transmission mechanism, and the bracket is movably arranged on the support in the transverse direction by the transmission mechanism.

In some embodiments, the transmission mechanism includes an electric motor, a driving wheel, a driven wheel, and a transmission belt sleeving outsides of the driving wheel and the driven wheel, the electric motor is arranged on the support, the driving wheel is arranged on an output shaft of the electric motor, the driven wheel is arranged on the support, and the transmission belt is in driving connection with the bracket.

In some embodiments, a connection member is arranged on the bracket, and the connection member is fixedly connected with the transmission belt.

In some embodiments, the transmission belt includes a synchronous belt, and the connection member is provided with a slot cooperating with a tooth of the synchronous belt in an insertion manner.

In some embodiments, a guide structure is arranged between the bracket and the support.

In some embodiments, the guide structure includes a guide rail and a guide groove cooperating with the guide rail, one of the guide rail and the guide groove is arranged on the bracket, and the other one of the guide rail and the guide groove is arranged on the support.

In some embodiments, the bracket is provided with a placing opening corresponding to the test window, the carrier is removably arranged at the placing opening, and the placing opening avoids the heating device.

A first shielding member is circumferentially provided on the support, the first shielding member shields circumferential outsides of the test window and the reagent loading device, the first shielding member is provided with an opening for avoiding the bracket and the carrier, the heating device includes a heating assembly, a driving assembly for driving the heating assembly to move, and a second shielding member arranged on a side of the heating assembly, and when the driving assembly drives the heating assembly to move to make the carrier located at the test position, the second shielding member shields the opening.

In some embodiments, the test device includes a heating cover arranged at the test window, and a test assembly arranged on the heating cover, and when the carrier is lifted to the test position cooperating with the heating cover in the butting manner, a part of the test assembly is located directly above a reagent hole of the carrier.

According the other aspect of the present invention, a carrier loading method for amplifying and testing is provided. The carrier is loaded by the above amplifying and testing device, and the carrier loading method for amplifying and testing includes: placing the carrier on the bracket of the reagent loading device; moving the bracket in the transverse direction, and stopping moving the bracket when the bracket transfers the carrier to the storage position below the test window of the support; and moving the heating device in the vertical direction, where the heating device lifts the carrier at the storage position, and stopping moving the heating device when the heating device lifts the carrier to the test position cooperating with the test device in the butting manner.

According to the technical solution of the present invention, the amplifying and testing device includes: the support, the test device, the reagent loading device and the heating device. The support is provided with the test window. The test device is arranged on the support and located at the test window, to test a reagent through the test window. The reagent loading device includes a bracket and a carrier removably arranged on the bracket, and the bracket is movably arranged on the support in the transverse direction. The reagent can be accommodated in the carrier. The carrier moves along with the bracket, such that the carrier has the storage position located below the test window and the loading position moving out of the support. The carrier accommodating the reagent is conveyed from the outside of the amplifying and testing device to the bracket, and the carrier accommodating the reagent moves along with the bracket between the storage position and the loading position. The heating device is movably arranged on the support in the vertical direction and can drive the carrier to lift, so as to lift the carrier to the test position cooperating with the test device in the butting manner. In this way, the carrier located at the test position can reduce a distance between the heating device and the carrier accommodating the reagent, such that the reagent in the carrier can be better heated to achieve a purpose of amplification, and in this case, the carrier is located at the test position, and the test device can test the reagent in the carrier, to estimate an amplification effect of the reagent. In this way, the test device, the reagent loading device and the heating device are vertically arranged by the support, such that an area occupied by the amplifying and testing device is reduced, a structure is compact, a space use rate is improved, and a plurality of amplifying and testing devices can be arranged side by side and implement processing in parallel, so as to achieve a target of high throughput. Thus, the technical solution of the invention effectively solves the problem of a large area occupied by the amplifying and testing device in the related art.

### Brief Description of the Drawings

The accompanying drawings of the specification forming a part of the invention serve to provide a further understanding of the present invention, and the illustrative embodiments of the present invention and the description of the illustrative embodiments serve to explain the present invention and are not to be construed as unduly limiting the present invention. In the drawings:
Fig. 1 is a schematic front view of a carrier of a reagent loading device at a loading position according to an embodiment of an amplifying and testing device in the present invention;
Fig. 2 is a schematic front view of the carrier of the reagent loading device of the amplifying and testing device in Fig. 1 at a storage position;
Fig. 3 is a schematic front view of the carrier of the reagent loading device of the amplifying and testing device in Fig. 1 at a test position;
Fig. 4 is a schematic diagram of a three-dimensional structure of the carrier of the reagent loading device of the amplifying and testing device in Fig. 1 at the loading position;
Fig. 5 is an enlarged partial diagram at portion A of the amplifying and testing device in Fig. 4;
Fig. 6 is a schematic top view of a support of the amplifying and testing device in Fig. 1;
Fig. 7 is a schematic front view of a test device of the amplifying and testing device in Fig. 1;
Fig. 8 is a schematic bottom view of a three-dimensional structure of the reagent loading device of the amplifying and testing device in Fig. 1;
Fig. 9 is a schematic diagram of a three-dimensional structure of the reagent loading device of the amplifying and testing device in Fig. 1;
Fig. 10 is an enlarged partial diagram at portion B of the reagent loading device of the amplifying and testing device in Fig. 9;
Fig. 11 is a schematic diagram of a three-dimensional structure of the reagent loading device of the amplifying and testing device in Fig. 1 from another viewing angle;
Fig. 12 is a schematic diagram of a three-dimensional structure of the amplifying and testing device in Fig. 1 without a test device and a first shielding member;
Fig. 13 is a schematic diagram of a three-dimensional structure of the amplifying and testing device in Fig. 12 with the first shielding member mounted;
Fig. 14 is a schematic diagram of a three-dimensional structure of a frame assembly of a heating device of the amplifying and testing device in Fig. 1;
Fig. 15 is a schematic front view of a linear motor and an insert of the frame assembly of the heating device in Fig. 14;
Fig. 16 is a schematic top view of a lifting plate of the heating device in Fig. 14;
Fig. 17 is a schematic top view of insertion cooperation between a lifting plate of the heating device in Fig. 14 and an insert;
Fig. 18 is a schematic front view of the heating device in Fig. 14 without an outer frame; and
Fig. 19 is a flowchart of a carrier loading method for amplifying and testing according to the present invention.

The above-mentioned figures include the following reference numerals:
100. support; 110. test window; 120. first shielding member; 130. opening;
200. test device; 210. heating cover; 220. test assembly;
300. reagent loading device; 310. bracket; 320. carrier; 340. connection member; 350. slot; 360. placing opening; 380. transmission mechanism; 381. electric motor; 382. driving wheel; 383. driven wheel; 384. synchronous belt;
400. heating device; 410. heating assembly; 420. driving assembly; 430. second shielding member;
500. guide structure; 510, guide rail; 520. guide groove;
10. outer frame; 11. top plate; 12. bottom plate; 13. first support member; 14. second support member;
20. inner frame; 21. third support member; 22. fourth support member;
30. lifting frame member; 31. first support guide member; 32. second support guide member; 33. bearing beam; 331. first bearing section; 332. second bearing section; 333. third bearing section; 34. support plate; 35. first baffle; 36. second baffle; 37. lifting plate; 371. connection hole; 372. first circular hole section; 373. second circular hole section; 38. first elastic member; 39. second elastic member;
41. output shaft; 42. insert; 421. connection portion; 422. upper block convex portion; 423. lower block convex portion; 424. slot;
51. trigger member; and 52. position detection member.

### Detailed Description of the Embodiments

The technical solutions of the embodiments of the present invention are clearly and completely described below with reference to the drawings. Apparently, the described embodiments are merely some embodiments rather than all embodiments of the present invention. The following description of at least one illustrative embodiment is merely illustrative in nature and in no way serves as any limitation of the present invention and its application or uses. Based on the embodiments of the present invention, other various embodiments obtained by those of ordinary skill in the art without making creative efforts fall within the scope of protection of the present invention.

It is to be noted that the terms used herein are for the purpose of describing detailed implementation modes only and are not intended to be limiting of the illustrative implementation modes in accordance with the invention. As used herein, the singular is intended to include the plural unless the context clearly dictates, and furthermore, it is to be understood that the terms "include" and/or "comprise", when used in this specification, specify the presence of features, steps, operations, devices, components, and/or combinations thereof.

Relative arrangements of components and steps, numerical expressions, and numerical values set forth in these embodiments do not limit the scope of the present invention unless otherwise specified. Moreover, it should be understood that for the convenience of description, sizes of various parts shown in the drawings are not drawn according to an actual scale relation. Techniques, methods, and apparatuses known to those of ordinary skill in the related art may not be discussed in detail, but should be considered part of the specification where appropriate. In all examples shown and discussed herein, any particular value should be interpreted as exemplary only, and not as limiting. Accordingly, other examples of illustrative embodiments may have different values. It should be noted that similar numerals and letters denote similar items in the following drawings. Therefore, once an item is defined in one drawing, it does not need to be further discussed in subsequent drawings.

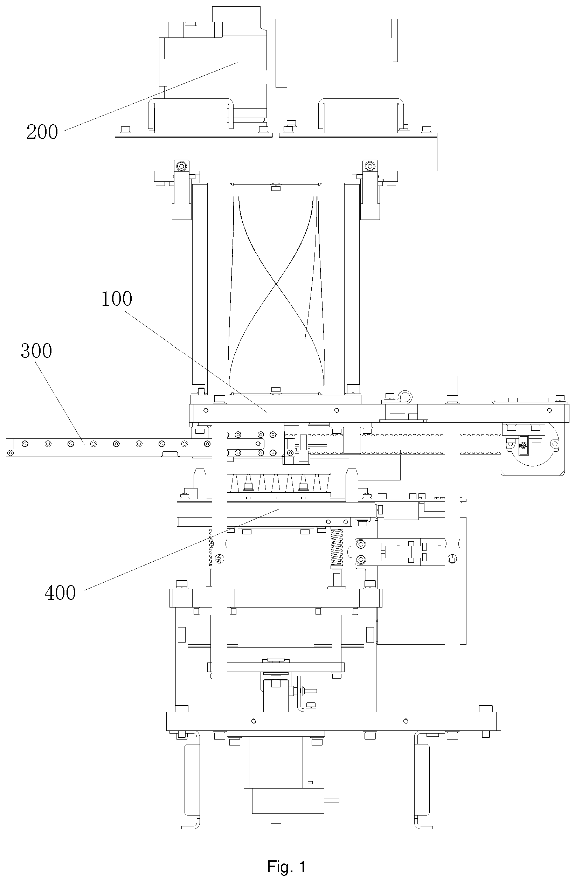

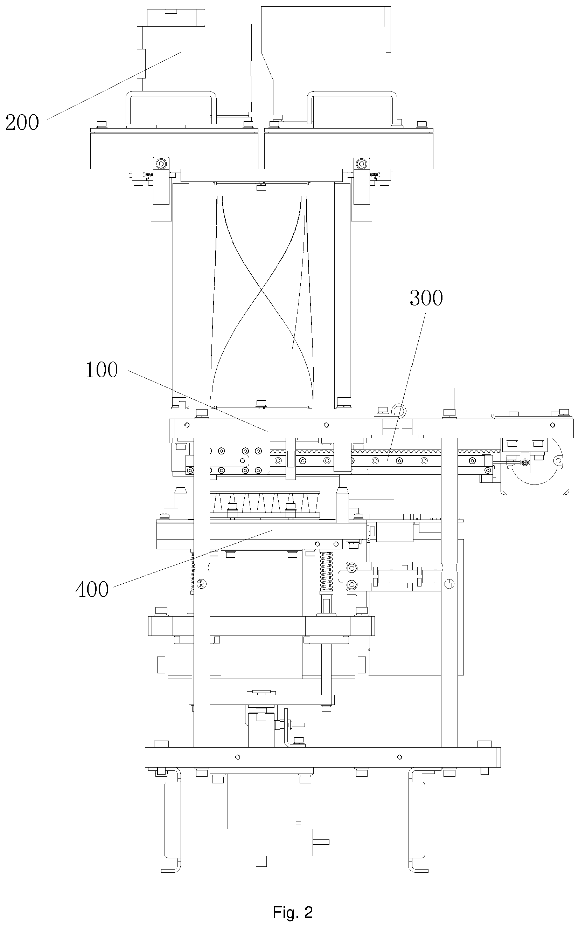

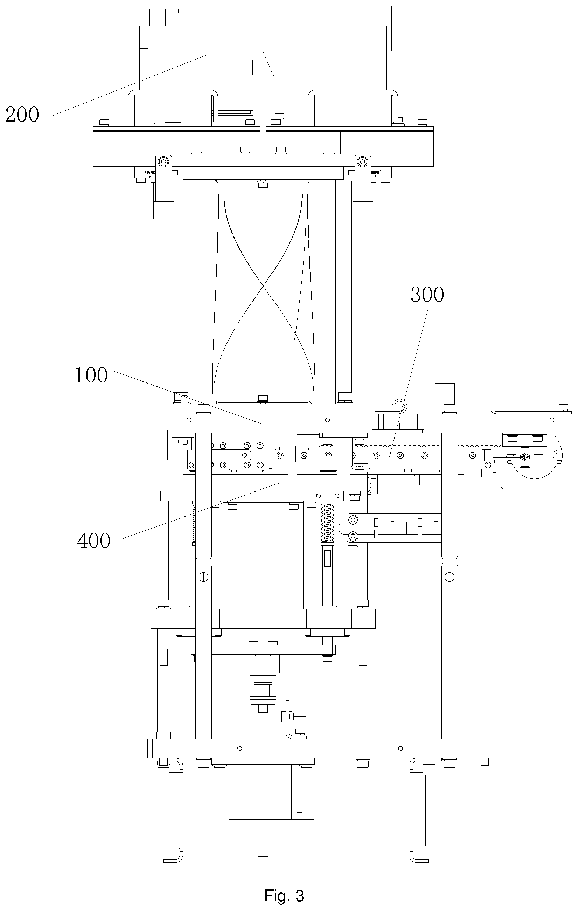

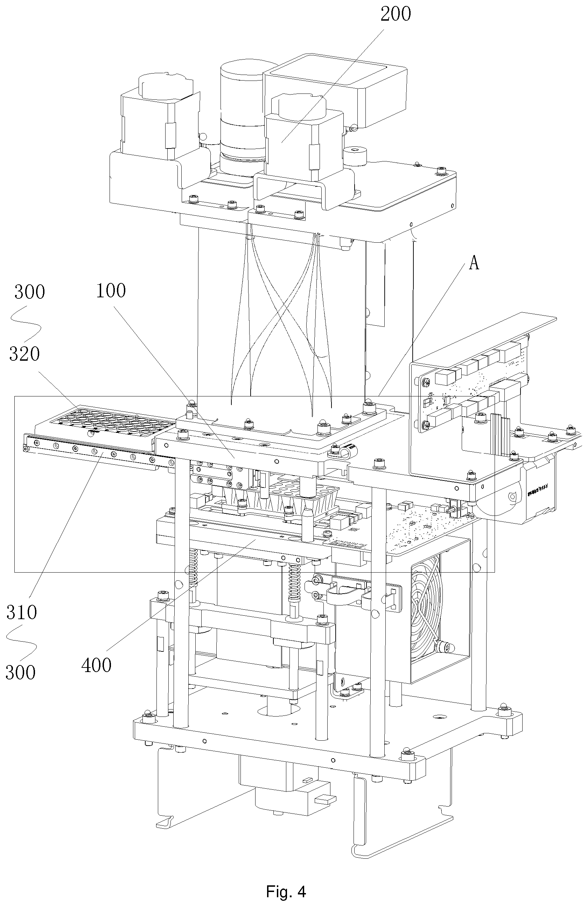

As shown in Figs. 1-5, an amplifying and testing device in an embodiment includes a support 100, a test device 200, a reagent loading device 300 and a heating device 400. The support 100 is provided with a test window 110. The test device 200 is arranged on the support 100 and located at the test window 110. The reagent loading device 300 includes a bracket 310 and a carrier 320 removably arranged on the bracket 310, and the bracket 310 is movably arranged on the support 100 in a transverse direction. The carrier 320 moves along with the bracket 310, such that the carrier 320 has a storage position located below the test window 110 and a loading position moving out of the support 100. The heating device 400 is movably arranged on the support 100 in a vertical direction and capable of driving the carrier 320 to lift, so as to lift the carrier 320 to a test position cooperating with the test device 200 in a butting manner.

According to the technical solution of the embodiment, the amplifying and testing device includes the support 100, the test device 200, the reagent loading device 300 and the heating device 400. The support 100 is provided with the test window 110. The test device 200 is arranged on the support 100 and located at the test window 110, to test a reagent through the test window 110. The reagent loading device 300 includes the bracket 310 and the carrier 320 removably arranged on the bracket 310, and the bracket 310 is movably arranged on the support 100 in the transverse direction. The reagent can be accommodated in the carrier 320. The carrier 320 moves along with the bracket 310, such that the carrier 320 has the storage position located below the test window 110 and the loading position moving out of the support 100. The carrier 320 accommodating the reagent is conveyed from the outside of the amplifying and testing device to the bracket 310, and the carrier 320 accommodating the reagent moves along with the bracket 310 between the storage position and the loading position. The heating device 400 is movably arranged on the support 100 in a vertical direction and capable of driving the carrier 320 to lift, so as to lift the carrier 320 to a test position cooperating with the test device 200 in the butting manner. In this way, the carrier 320 located at the test position can reduce a distance between the heating device 400 and the carrier 320 accommodating the reagent, such that the reagent in the carrier 320 can be better heated to achieve a purpose of amplification, and in this case, the carrier 320 is located at the test position, and the test device 200 can test the reagent in the carrier 320, to estimate an amplification effect of the reagent. In this way, the test device 200, the reagent loading device 300 and the heating device 400 are vertically arranged by the support 100, such that an area occupied by the amplifying and testing device is reduced, a structure is compact, a space use rate is improved, and a plurality of amplifying and testing devices can be arranged side by side and implement processing in parallel, so as to achieve a target of high throughput. Thus, the technical solution of the embodiment effectively solves the problem of a large area occupied by the amplifying and testing device in the related art.

It should be noted that cooperating in a butting manner may be contact cooperating or cooperating with a certain gap.

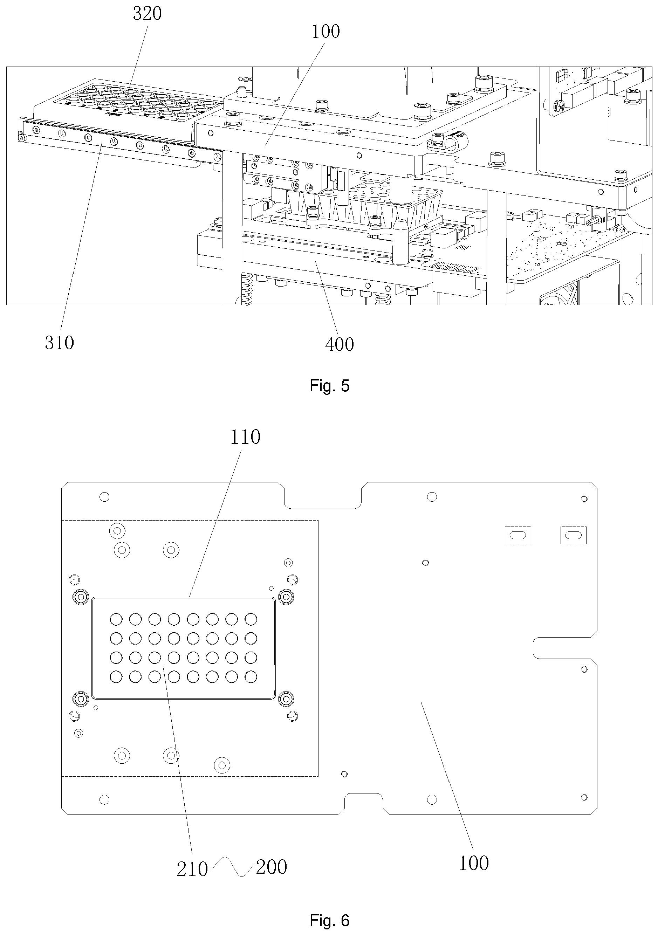

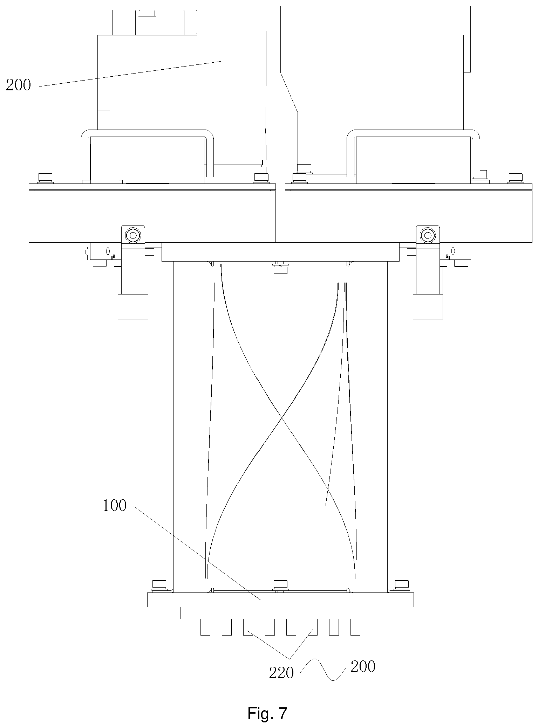

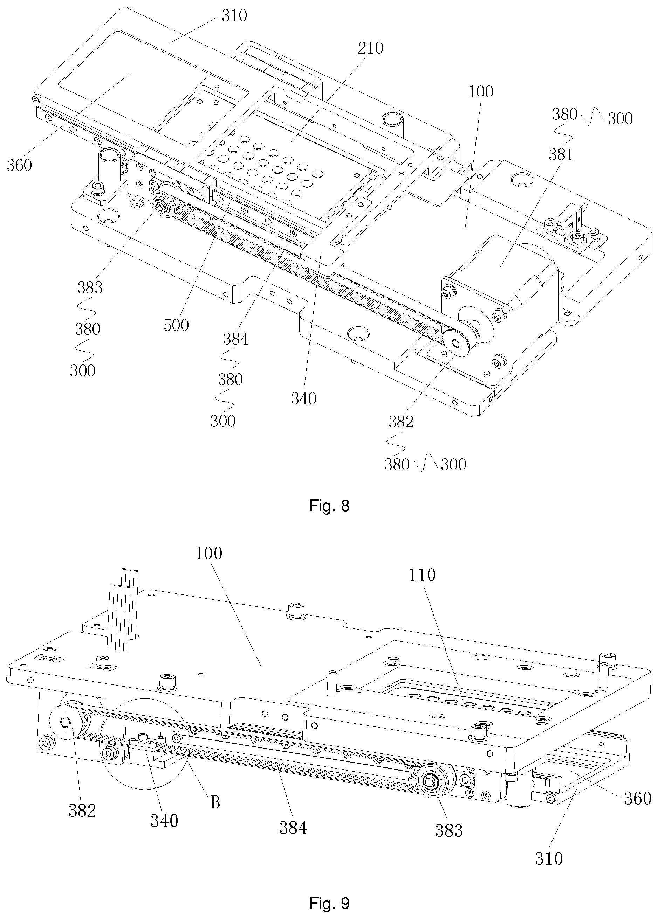

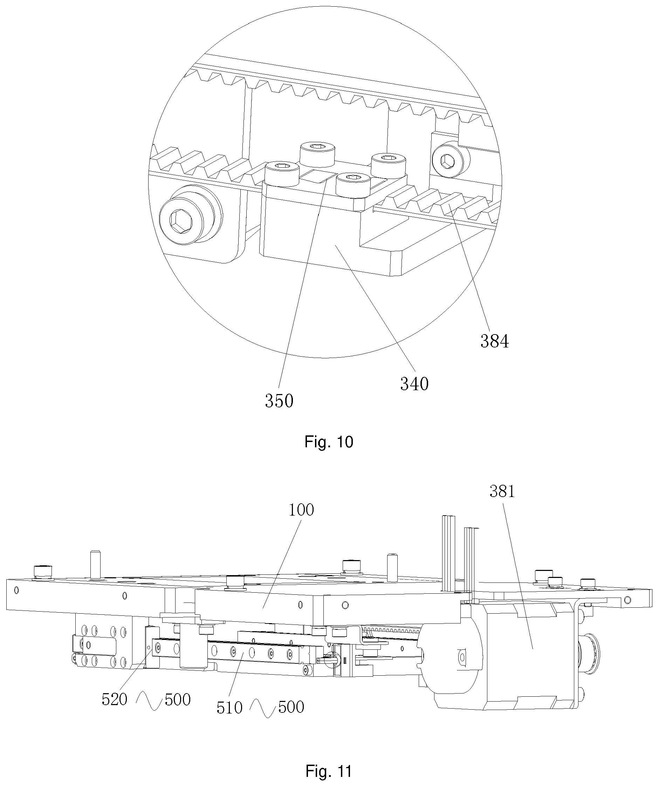

As shown in Figs. 5-11, the reagent loading device 300 further includes a transmission mechanism 380, and the bracket 310 is movably arranged on the support 100 in the transverse direction by the transmission mechanism 380. The transmission mechanism 380 facilitates movement of the carrier 320 between the storage position and the loading position driven by the bracket 310, which improves an automation level of the amplifying and testing device and reduces manual labor. In this embodiment, the bracket 310 is provided with a trigger member, and the support 100 is provided with a sensing member in sensing cooperation with the trigger member. After the bracket 310 drives the carrier 320 to reach the storage position, the trigger member triggers the sensing member, and the sensing member transmits information that the carrier 320 reaches the storage position to a controller for subsequent operation.

It is to be noted that the transmission mechanism 380 may be a belt transmission mechanism, a rack and pinion transmission mechanism, a screw nut transmission mechanism, or a crank slider transmission mechanism.

As shown in Figs. 5-11, the transmission mechanism 380 includes an electric motor 381, a driving wheel 382, a driven wheel 383, and a transmission belt sleeving outsides of the driving wheel 382 and the driven wheel 383. The electric motor 381 is arranged on the support 100, the driving wheel 382 is arranged on an output shaft of the electric motor 381, the driven wheel 383 is arranged on the support 100, and the transmission belt is in driving connection with the bracket 310. The electric motor 381 rotates to drive the driving wheel 382 to rotate. The driving wheel 382 drives the driven wheel 383 to rotate by the transmission belt. The transmission belt drives the bracket 310 to move. The bracket 310 drives the carrier 320 to move between the storage position and the loading position. The transmission belt drives the bracket 310, and the bracket 310 drives the carrier 320, such that the carrier 320 can smoothly move in the transverse direction between the storage position and the loading position. In the embodiment, the support 100 includes a frame assembly for the heating device and an upper frame arranged above the frame assembly.

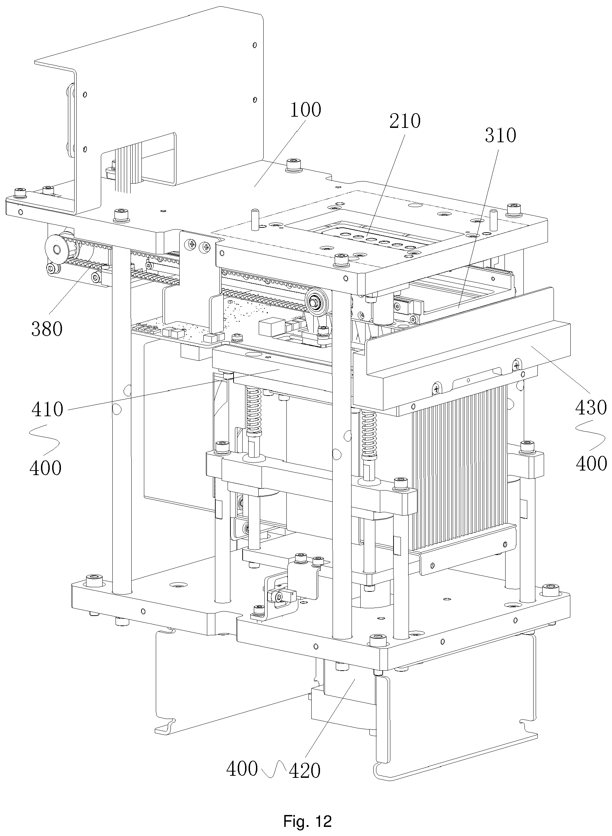

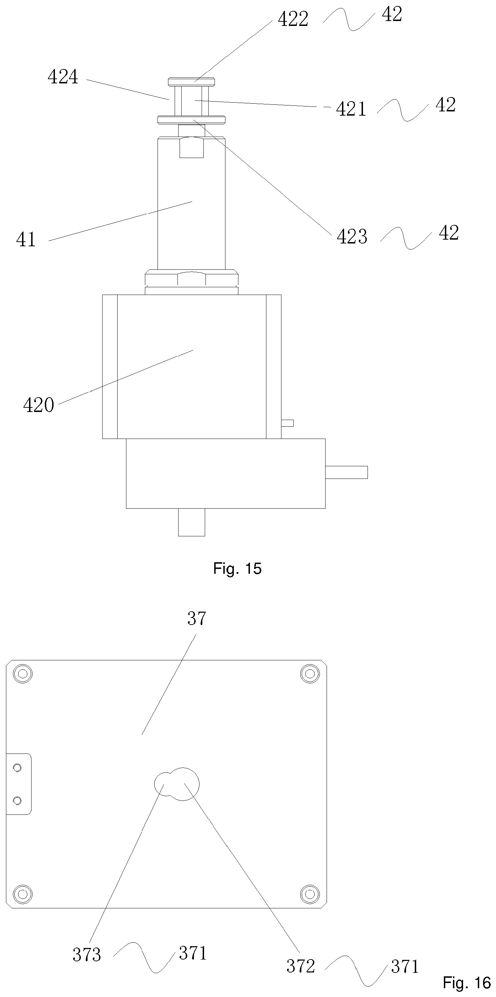

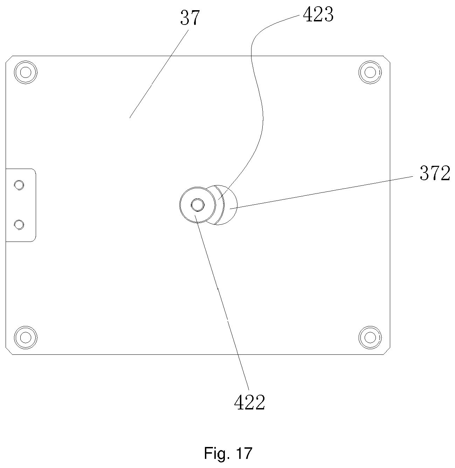

As shown in Figs. 12-18, the frame assembly of the heating device of the embodiment includes an outer frame 10, an inner frame 20 and a lifting frame member 30. The outer frame 10 includes a top plate 11 and a bottom plate 12 that are spaced, and a first support member 13 and a second support member 14 arranged between the top plate 11 and the bottom plate 12. The top plate 11, the bottom plate 12, the first support member 13 and the second support member 14 define a lifting space. The test window 110 is arranged on the top plate 11 of the frame assembly. The bracket 310 and the electric motor 381 are both arranged on the top plate 11. The driving wheel 382, the driven wheel 383 and the transmission belt are arranged below the top plate 11.

As shown in Figs. 5-11, a connection member 340 is arranged on the bracket 310, and the connection member 340 is fixedly connected with the transmission belt. The arrangement of the connection member 340 facilitates the driving connection between the transmission belt and the bracket 310, to make the bracket 310 move along with the transmission belt by the connection member 340. In this way, when the electric motor 381 in the embodiment rotates clockwise, the driving wheel 382 drives the transmission belt to move, such that the transmission belt drives the connection member 340 to move to drive the carrier 320 on the bracket 310 to move to the loading position. When the electric motor 381 rotates anticlockwise, the driving wheel 382 drives the transmission belt to move, such that the transmission belt drives the connection member 340 to move to drive the carrier 320 on the bracket 310 to move to the storage position. Moreover, fixed connection between the connection member 340 and the transmission belt makes the connection member 340 move synchronously with the transmission belt, so as to avoid relative movement between the connection member 340 and the transmission belt, and further to improve stability of movement of the carrier 320 driven by the bracket 310.

As shown in Figs. 5-11, the transmission belt includes a synchronous belt 384, and the connection member 340 is provided with a slot 350 cooperating with a tooth of the synchronous belt 384 in an insertion manner. The tooth of the synchronous belt 384 is inserted into the slot 350, which facilitates assembly of the synchronous belt 384 and the connection member 340. The carrier 320 is driven by the bracket 310 to move more stably in a process of cooperation between the tooth of the synchronous belt 384 and the slot 350. In the embodiment, the tooth of the synchronous belt 384 is inserted into the slot 350, and the synchronous belt 384 and the connection member 340 are fastened by screws, so as to improve the reliability of fixed connection between the connection member 340 and the transmission belt.

As shown in Figs. 5-11, a guide structure 500 is arranged between the bracket 310 and the support 100. The bracket 310 can move in a guide direction of the guide structure 500 by the arrangement of the guide structure 500, such that the bracket 310 can move more stably, such that the carrier 320 can be driven by the bracket 310 to move smoothly in the guide direction. In the embodiment, the guide direction of the guide structure 500 is transverse, and the bracket 310 drives the carrier 320 to move in the transverse direction. The bracket 310 is a rectangular plate, the connection member 340 is arranged on a shorter side of the rectangular plate facing the electric motor 381, and two guide structures 500 are symmetrically arranged on two longer sides of the rectangular plate.

As shown in Figs. 5-11, the guide structure 500 includes a guide rail 510 and a guide groove 520 cooperating with the guide rail 510. The guide rail 510 is arranged on the bracket 310, and the guide groove 520 is arranged on the support 100. Structures of the guide rail 510 and the guide groove 520 are simple, which facilitates machining and mounting of the guide structure 500. Furthermore, linear movement of the bracket 310 can be conveniently limited by of the arrangement of the guide rail 510 and the guide groove 520. In the embodiment, it is convenient for the bracket 310 to drive the carrier 320 to move horizontally in the transverse direction. A guide block is arranged on a lower surface of the top plate, and the guide groove 520 is provided on the guide block. The bracket 310 is a rectangular plate, the two guide rails 510 are arranged on two longer sides of the rectangular plate. The two guide blocks are symmetrically arranged on outer sides of the two longer sides of the rectangular plate. Each guide rail 510 cooperates with the guide groove 520 on the corresponding guide block in a guide manner.

In an embodiment not shown in the figures, the guide groove is provided on the bracket, and the guide rail is arranged on the support.

As shown in Figs. 1-11, the bracket 310 is provided with a placing opening 360 corresponding to the test window 110. The carrier 320 is removably arranged at the placing opening 360. The bracket 310 can support the carrier 320 conveniently by the arrangement of the placing opening 360, such that the bracket 310 can drive the carrier 320 to move. When the heating device 400 moves in the vertical direction, the placing opening 360 avoids the heating device 400, such that the heating device 400 can pass through the placing opening 360, to drive the carrier 320 to lift. In the embodiment, the placing opening 360 is provided with a sinking platform, and the sinking platform supports and limits the placed carrier 320, such that the carrier 320 moves more stably.

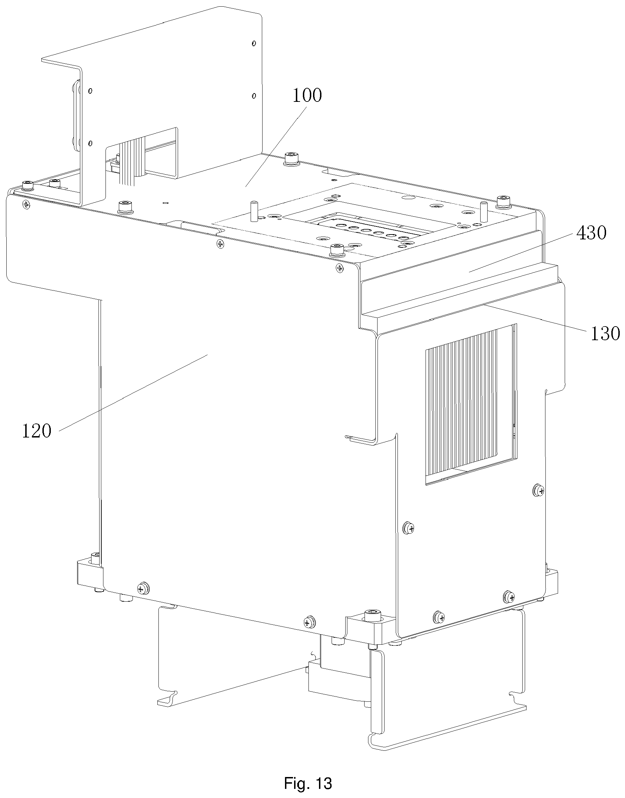

As shown in Figs. 12 and 13, a first shielding member 120 is arranged on the support 100. The first shielding member 120 circumferentially shields outsides of the test window 110 and the reagent loading device 300. The first shielding member 120 can shield external light from entering the reagent of the carrier 320. The first shielding member 120 is provided with an opening 130 for avoiding the bracket 310 and the carrier 320, such that the bracket 310 can pass through the opening 130 of the first shielding member 120 and move in the transverse direction, then the bracket 310 can extend out of the first shielding member 120, and the carrier 320 outside the amplifying and testing device can be placed at the placing opening 360 of the bracket 310. The heating device 400 includes a heating assembly 410, a driving assembly 420 for driving the heating assembly 410 to move, and a second shielding member 430 arranged on a side of the heating assembly 410. When the driving assembly 420 drives the heating assembly 410 to move to make the carrier 320 located at the test position, the second shielding member 430 shields the opening 130. In this way, when the carrier 320 is located at a position other than the test position, the opening 130 of the first shielding member 120 can avoid the movement of the bracket 310. When the carrier 320 is located at the test position, the second shielding member 430 shields the opening 130, such that the carrier 320 accommodating the reagent is in a sealed environment, and external light is prevented from entering the carrier 320 accommodating the reagent. The second shielding member 430 can move along with the heating assembly 410, and can seal an amplification reaction environment of the reagent in the carrier 320 without providing an additional structure, so as to prevent the reagent from being influenced by external light during amplification or test.

Specifically, the second shielding member 430 is a step block. In order to avoid interference between the step block and a corresponding structure, a first avoiding step surface is provided on an upper portion of a rear surface of the step block facing the opening 130, and a second avoiding step surface is provided on a front surface of the step block facing away from the opening 130.

As shown in Figs. 12-18, the frame assembly of the heating device of the embodiment includes an outer frame 10, an inner frame 20 and a lifting frame member 30. The outer frame 10 includes a top plate 11 and a bottom plate 12 that are spaced, and a first support member 13 and a second support member 14 arranged between the top plate 11 and the bottom plate 12. The top plate 11, the bottom plate 12, the first support member 13 and the second support member 14 define a lifting space. The inner frame 20 includes a third support member 21 and a fourth support member 22 spaced between the bottom plate 12 and the top plate 11. The lifting frame member 30 is liftably located in the lifting space. The lifting frame member 30 includes a lifting plate 37 liftably penetrating the third support member 21 and the fourth support member 22, a first support guide member 31 penetrating the third support member 21, and a second support guide member 32 penetrating the fourth support member 22. The first support guide member 31 and the second support guide member 32 are both connected with the lifting plate 37. The third support member 21, the fourth support member 22 and the lifting plate 37 define a placing space. The driving assembly 420 is arranged on the bottom plate 12 and is in driving connection with the lifting plate 37.

According to the technical solution of the embodiment, the frame assembly of the heating device includes the outer frame 10, the inner frame 20 and the lifting frame member 30. The outer frame 10 includes the top plate 11 and the bottom plate 12 that are spaced, and the first support member 13 and the second support member 14 arranged between the top plate 11 and the bottom plate 12. The arrangement of top plate 11 facilitates cooperation with an apparatus above the heating device. The top plate 11, the bottom plate 12, the first support member 13 and the second support member 14 define the lifting space. The inner frame 20 includes the third support member 21 and the fourth support member 22 spaced between the bottom plate 12 and the top plate 11. The lifting frame member 30 is liftably located in the lifting space. The lifting frame member 30 includes the lifting plate 37 liftably penetrating the third support member 21 and the fourth support member 22, the first support guide member 31 penetrating the third support member 21, and the second support guide member 32 penetrating the fourth support member 22. In this way, the first support guide member 31 and the second support guide member 32 can play a guide role when the lifting frame member 30 is lifted, such that the lifting frame member 30 can be lifted more stably. The first support guide member 31 and the second support guide member 32 are both connected with the lifting plate 37. The third support member 21, the fourth support member 22 and the lifting plate 37 define the placing space. The driving assembly 420 is arranged on the bottom plate 12 and is in driving connection with the lifting plate 37. The heating assembly 410 may be placed in the placing space, such that the first support guide member 31 and the second support guide member 32 can support the heating assembly 410 while implementing guiding, and then the heating assembly is supported by the lifting frame member 30. The driving assembly 420 drives the lifting plate 37 to lift, such that the lifting frame member 30 drives the heating assembly 410 to lift. The carrier accommodating the reagent may be placed on the top plate 11, the lifting frame member 30 drives the heating assembly 410 to rise, and the heating assembly 410 controls a temperature of the carrier accommodating the reagent on the top plate 11. In this way, the inner frame 20 is nested in the outer frame 10, and the lifting frame member 30 is nested in the inner frame 20. Such a mutually nested structure makes a structure of the frame assembly of the heating device compact, so as to reduce a size and an occupied area of the heating device. Moreover, since the lifting frame member 30 is nested in the inner frame 20 for lifting, which fully use the lifting space in the vertical direction, horizontal movement of the heating assembly 410 in the related art is avoided, the area occupied by the heating device is reduced, and an area occupied by the amplifying and testing device having the heating device is reduced, such that an occupied area required for arranging a plurality of amplifying and testing devices is reduced, which is beneficial to realization of a high-throughput target.

As shown in Figs. 1-11, the test device 200 includes a heating cover 210 arranged at the test window 110, and a test assembly 220 arranged on the heating cover 210. When the carrier 320 is lifted to the test position cooperating with the heating cover 210 in the butting manner, a part of the test assembly 220 is located directly above a reagent hole of the carrier 320. In this way, the carrier 320 accommodating the reagent is lifted to cooperate the heating cover 210 in the butting manner, such that a top of the carrier 320 accommodating the reagent can be heated by the heating cover 210, and the reagent in the carrier 320 is heated more uniformly, which optimizes the amplification effect of the test device 200. Furthermore, since the part of the test assembly 220 is located directly above the reagent hole of the carrier 320, it is convenient for the test assembly 220 to test the amplification effect of the reagent in the carrier 320. In the embodiment, the carrier 320 is pushed away from the bracket 310, to make contact with the heating cover 210 when the carrier is at the test position.

In the embodiment, the test window 110 is a rectangular opening 130 penetrating an upper surface of the top plate and a lower surface of the top plate. The heating cover 210 is arranged on the lower surface of the top plate and covers the test window 110. The test device 200 further includes an optical fiber mounting structure arranged on the top plate. The optical fiber mounting structure includes a mounting base and a plurality of optical fiber mounting posts arranged on the mounting base. The optical fiber mounting posts test the reagent in the carrier 320 by using optical signals. The heating cover 210 is provided with a plurality of avoiding holes in a one-to-one correspondence to a plurality of reaction holes on the carrier 320, such that the optical fiber mounting posts of the test device 200 can extend into the avoiding holes of the heating cover 210 and test the reagents in the reaction holes of the carrier 320.

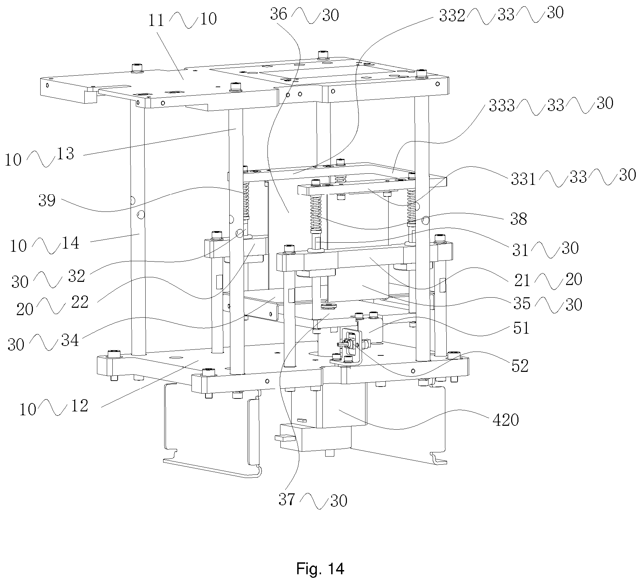

In the embodiment, the mounting base of the optical fiber mounting structure includes a positioning plate and a mounting lug. The mounting lug is arranged on a surface of the positioning plate facing the heating cover 210. The plurality of optical fiber mounting posts are arranged on the mounting lug. The mounting lug is a rectangular lug corresponding to the rectangular test window 110. The mounting lug extends into the test window 110, such that the plurality of optical fiber mounting posts on the mounting lug can extend into the avoiding holes in a one-to-one correspondence. After the mounting lug extends into the test window 110, the positioning plate can be connected with a portion of the top plate not provided with the test window 110, so as to limit a descending distance of the mounting lug and avoid damage to the heating cover 210 due to continuous descending of the mounting lug. As shown in Figs. 14-18, the first support member 13 is two first support rods. The two first support rods are connected with a side of the top plate 11 and a side of the bottom plate 12. The second support member 14 is two second support rods. The two second support rods are connected with the other side of the top plate 11 and the other side of the bottom plate 12. The two first support rods and the two second support rods are oppositely arranged. The third support member 21 includes a support transverse rod and two support vertical rods. An end of the support vertical rod is connected with the bottom plate 12, and the other end of the support vertical rod is connected with the support transverse rod. The first support guide member 31 penetrates the support transverse rod. The fourth support member 22 is arranged in the same way as the third support member 21. The third support member 21 is arranged parallel to the fourth support member 22. A support is provided on a lower surface of the bottom plate 12, to support the bottom plate 12, so as to provide a space for the arrangement of the driving assembly 420.

As shown in Figs. 14-18, the driving assembly 420 is a linear motor. The linear motor can directly convert electric energy into linear motion, such that the linear motor can conveniently drive the lifting plate 37 to lift. The lifting plate 37 is connected with an output shaft 41 of the linear motor. The linear motor can directly drive the lifting frame member 30 to lift by connection between the lifting plate 37 and the output shaft 41 of the linear motor, so as to avoid energy loss of the linear motor.

As shown in Figs. 14-18, the lifting plate 37 is provided with a connection hole 371, and the output shaft 41 is fixedly connected in the connection hole 371. This facilitates fixed connection between the output shaft 41 of the linear motor and the lifting plate 37, and operation is easy.

As shown in Figs. 14-18, the connection hole 371 includes a first circular hole section 372 and a second circular hole section 373 in communication with the first circular hole section 372. A diameter of the first circular hole section 372 is less than a diameter of the second circular hole section 373. The output shaft 41 is provided with an insert 42. The insert 42 can move from the first circular hole section 372 to a position cooperating with the second circular hole section 373 in an insertion manner. When the output shaft 41 of the electric motor needs to be connected with the lifting plate 37, the insert 42 of the output shaft 41 is inserted into the first circular hole section 372 of the connection hole 371, and then the insert 42 of the output shaft 41 is moved to the second circular hole section 373, such that the insert 42 of the output shaft 41 can be cooperated with the second circular hole section 373 in the insertion manner, and the output shaft 41 of the linear motor can be fixedly connected with the lifting plate 37 by the insert 42. Disassembly of the output shaft 41 of the electric motor and the lifting plate 37 is convenient by the arrangement of the insert 42, and maintenance and mounting of the electric motor are convenient.

It is to be noted that communication of the first circular hole section 372 and the second circular hole section 373 means that the first circular hole section 372 and the second circular hole section 373 partially overlap.

As shown in Figs. 14-18, the insert 42 includes a connection portion 421 connected with an end of the output shaft 41, and an upper block convex portion 422 and a lower block convex portion 423 arranged on the connection portion 421 at an interval. After the insert 42 of the output shaft 41 is inserted into the first circular hole section 372 of the connection hole 371, since a maximum surface area of the lower block convex portion 423 is greater than a cross-sectional area of the first circular hole section 372, the lower block convex portion 423 can support the lifting plate 37, and drive force of the electric motor is transmitted to the lifting plate 37 through the lower block convex portion 423 of the insert 42, to drive the lifting plate 37 to rise. A maximum surface area of the upper block convex portion 422 is less than the cross-sectional area of the first circular hole section 372, such that the upper block convex portion 422 of the insert 42 can be inserted into the first circular hole section 372 of the connection hole 371. When the insert 42 of the output shaft 41 is inserted into the second circular hole section 373, the electric motor drives the lifting plate 37 to descend. Since the maximum surface area of the upper block convex portion 422 is greater than a cross-sectional area of the second circular hole section 373, the upper block convex portion 422 can abut against an upper surface of the lifting plate 37 around the first circular hole section 372, so as to drive the lifting plate 37 to descend by the upper block convex portion 422 of the insert 42. The upper block convex portion 422, the lower block convex portion 423, and the connection portion 421 define a slot 424. The slot 424 can cooperate with the second circular hole section 373 in an insertion manner. A side wall of the slot 424 facilitates insertion cooperation with the second circular hole section 373 and abutting cooperation with the upper surface and the lower surface of the lifting plate 37. Moreover, the slot 424 facilitates machining of the upper block convex portion 422 and the lower block convex portion 423 of the insert 42.

It is to be noted that the cross-sectional area of the first circular hole section 372 refers to an area of a cross-sectional shape perpendicular to an axis of the first circular hole section 372, and the same applies to the cross-sectional area of the second circular hole section 373.

As shown in Figs. 14-18, the lifting frame member 30 further includes a bearing beam 33 floatably connected with the first support guide member 31 and the second support guide member 32. The bearing beam 33 is located above the inner frame 20. A first elastic member 38 is arranged between the bearing beam 33 and the first support guide member 31. A second elastic member 39 is arranged between the bearing beam 33 and the second support guide member 32. The first elastic member 38 and the second elastic member 39 can support the lifting frame member 30, so that the lifting frame member 30 can float. The heating assembly 410 may be placed on the bearing beam 33 of the lifting frame member 30, the carrier accommodating the reagent may be placed on the top plate 11, and the linear motor drives the bearing beam 33 of the lifting frame member 30 to drive the heating assembly 410 to rise, such that the heating assembly 410 abuts against the carrier accommodating the reagent and controls the temperature of the carrier. After the bearing beam 33 drives the heating assembly 410 to rise to a position abutting against the carrier accommodating the reagent, the bearing beam 33 can float, such that when the heating assembly 410 abuts against the carrier accommodating the reagent, the problem that the carrier or other devices abutting against the carrier upwardly are damaged due to an excessive abutting force applied to the carrier can be avoided, and buffering is achieved.

In the embodiment, the first support guide member 31 is two first support guide rods, and the second support guide member 32 is two second support guide rods. A first elastic member 38 sleeves an outer side of each first support guide rod, and a second elastic member 39 sleeves an outer side of each second support guide rod. The first elastic member 38 is preferably a first spring, and the second elastic member 39 is preferably a second spring. A portion of the first support guide rod corresponding to an inner part of the first spring has a smaller outer diameter, such that abrasion between the first support guide rod and the first spring can be reduced.

As shown in Figs. 14-18, the first support guide member 31 is provided with an upper stop member and a lower stop member that are spaced. The upper stop member is located above the bearing beam 33, and the first elastic members 38 are located between the bearing beam 33 and the lower stop member. The arrangement of the upper stop member prevents the bearing beam 33 from being separated from the first support guide member 31 caused by an upward elastic force of the first elastic members 38. The arrangement of the lower stop member stops the first elastic members 38 when the first elastic members 38 are pressed downwards by the bearing beam 33, such that a lower end of each first elastic member 38 can be mounted on the first support guide member 31. The bearing beam 33 can float up and down on the first support guide member 31 by the arrangements of the upper stop member, the lower stop member and the first elastic members 38. Similarly, the second support guide member 32 is provided with an upper stop member and a lower stop member that are the same as those arranged on the first support guide member 31, such that the bearing beam 33 can float up and down on the first support guide member 31 and the second support guide member 32.

In the embodiment, the top plate 11 is provided with the test window, the test device is arranged at the test window, and the carrier accommodating the reagent is placed below the test window. The heating assembly 410 is provided with an avoiding hole for avoiding a top end of the first support guide member 31 and a top end of the second support guide member 32. When the bearing beam 33 is lifted to make the heating assembly 410 abut against the lower surface of the top plate 11, the linear motor continuously drives the lifting frame member 30. In this case, the top end of the first support guide member 31 and the top end of the second support guide member 32 extend into the avoiding hole, such that the bearing beam 33 can float down.

As shown in Figs. 14-18, the lifting frame member 30 further includes a bearing beam 33 floatably connected with the first support guide member 31 and the second support guide member 32. The bearing beam 33 is located above the inner frame 20. The lifting frame member 30 further includes a support plate 34, a first baffle 35, and a second baffle 36. The support plate 34 is located between the first support guide member 31 and the second support guide member 32 and below the bearing beam 33. A first side of the support plate 34 is connected to a portion of the bearing beam 33 by the first baffle 35, and a second side of the support plate 34 is connected to another portion of the bearing beam 33 by the second baffle 36. The support plate 34 is connected to the bearing beam 33 by the first baffle 35 and the second baffle 36, and moves up and down synchronously with the bearing beam 33. The support plate 34, the first baffle 35, the second baffle 36 and the top plate 11 define a placing space. After the heating assembly 410 is placed in the placing space, the support plate 34 can support a bottom of the heating assembly 410, and the bearing beam 33 can support an upper edge of the heating assembly 410, such that structural strength of the lifting frame member 30 can be improved, and a bearing capacity of the lifting frame member 30 is improved.

As shown in Figs. 14-18, the bearing beam 33 includes a first bearing section 331 sleeving the top end of the first support guide member 31, a second bearing section 332 sleeving the top end of the second support guide member 32, and a third bearing section 333 connected with a first end of the first bearing section 331 and a first end of the second bearing section 332. The arrangement of the first bearing section 331, the second bearing section 332, and the third bearing section 333 can improve the structural strength of the bearing beam 33, thereby improving the bearing capacity of the bearing beam 33. An opening is formed between a second end of the first bearing section 331 and a second end of the second bearing section 332, such that the heating assembly 410 can be placed on the bearing beam 33 through the opening.

As shown in Figs. 14-18, the lifting frame member 30 is provided with a trigger member 51, and the bottom plate 12 is provided with a position detection member 52 in sensing cooperation with the trigger member 51. The lifting frame member 30 moves downward, and when the trigger member 51 triggers the position detection member 52 on the bottom plate 12, the position detection member 52 sends a signal to a controller, such that the controller knows that the lifting frame member 30 has been lowered to a preset position. The arrangement of the trigger member 51 and the position detection member 52 improves an automation level of the heating device. A movement stroke of the linear motor controls a distance by which the lifting frame member 30 moves upwards.

As shown in Figs. 14-18, a placing channel is formed between the first support member 13 and the second support member 14 and between the third support member 21 and the fourth support member 22. The placing channel is in communication with the placing space. The heating assembly 410 of the heating device can be conveniently placed into the frame assembly of the heating device through the placing channel.

In the embodiment, the trigger member 51 is a bent plate arranged on the lifting plate 37 and bent downwards, and the position detection member 52 is an optocoupler assembly arranged on an upper surface of the bottom plate 12.

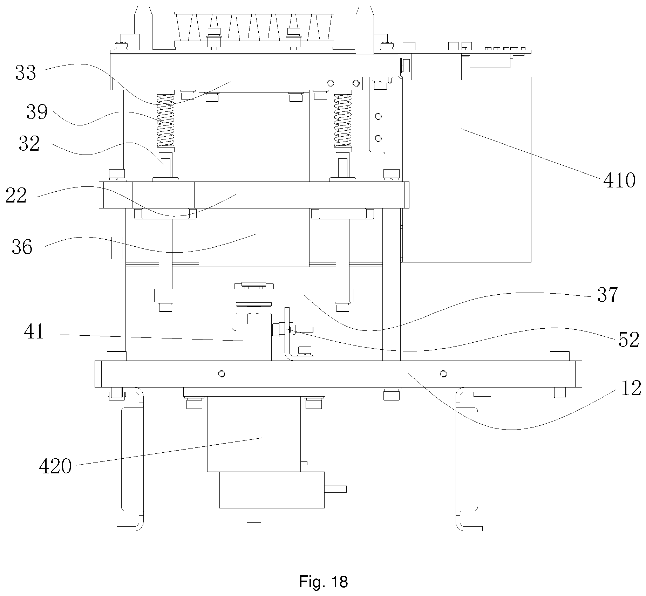

The invention further provides a heating device, as shown in Fig. 18, the heating device includes a frame assembly and a heating assembly 410 arranged on the frame assembly. The frame assembly is the frame assembly of the heating device described above. Since the frame assembly of the heating device can solve the problem of a large area occupied by a heating device in the related art, the heating device having the frame assembly can solve the same technical problem.

In the embodiment, the heating assembly 410 includes a perforated plate base, a heat sink arranged below the perforated plate base, a circuit board, and a vapor chamber. A top of the heat sink is provided with a convex edge, and the convex edge surrounds a circumferential outer side of the top of the heat sink, such that an upper surface of the bearing beam of the frame assembly of the heating device 400 can abut against the convex edge of the heat sink, so as to receive the heat sink and drive the heat sink to lift. When the carrier 320 is in the storage position, the carrier 320, the test window 110, the perforated plate base, the heating cover 210, and the heating assembly 410 are in the same vertical direction.

A specific workflow of the embodiment is as follows. The carrier 320 filled with the reagent is subjected to film sealing in a film sealing mechanism, and then is transferred into a nucleic acid amplification region by a ferry mechanism. A gripper transfers the carrier 320 filled with the reagent from the ferry mechanism in the nucleic acid amplification region to the bracket 310 of the reagent loading device 300. The reagent loading device 300 drives the carrier 320 to move to the storage position. When the heating device 400 vertically rises to receive the carrier 320 and moves upward for a certain distance, the carrier 320 reaches the test position. The lifting frame floats, such that the first elastic member and the second elastic member are compressed to a specified height, the linear motor 381 stops pushing, the heating device 400 and the heating cover 210 are heated and cooled according to a preset program, and the reagent in the carrier 320 starts to be amplified, so as to guarantee progress of the amplification reaction. Meanwhile, the test device 200 collects a fluorescence signal in the reagent in the carrier 320 by using an optical fiber in the optical fiber mounting posts, to form a test result. After amplification and test are completed, the heating device 400 descends, and the carrier 320 descends along with the heating device 400 for a certain distance to the storage position. In this case, the carrier 320 is received by the bracket 310 and separated from the heating device 400. The carrier 320 is driven by the bracket 310 to move transversely, such that the carrier 320 is located at the loading position, and the gripper grabs and moves the carrier 320 to a recovery mechanism, to recover the carrier 320, so as to complete an amplifying and testing process of the carrier 320.

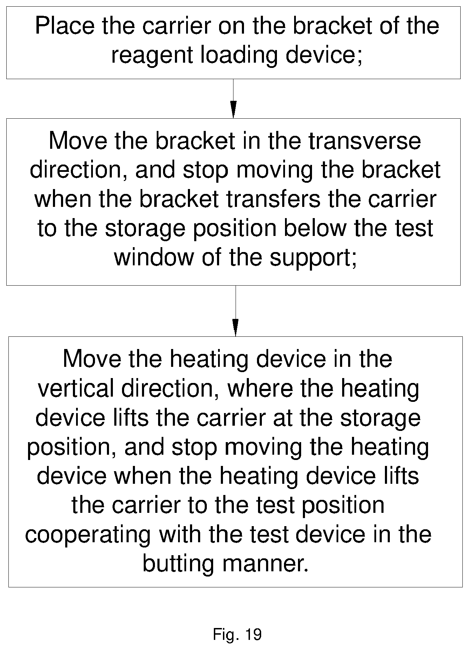

As shown in Fig. 19, the invention further provides a carrier loading method for amplifying and testing. The carrier is loaded by the above amplifying and testing device, and the carrier loading method for amplifying and testing includes: place the carrier 320 on the bracket 310 of the reagent loading device 300; move the bracket 310 in the transverse direction, and stop moving the bracket 310 when the bracket 310 transfers the carrier 320 to the storage position below the test window 110 of the support 100; and move the heating device 400 in the vertical direction, where the heating device 400 lifts the carrier 320 at the storage position, and stop moving the heating device 400 when the heating device 400 lifts the carrier 320 to the test position cooperating with the test device 200 in the butting manner. Since the amplifying and testing device can solve the problem of a large area occupied by an amplifying and testing device in the related art, the carrier loading method for amplifying and testing used for loading the carrier by the amplifying and testing device can solve the same technical problem.

In the description of the present invention, it is to be understood that the directional terms such as "front", "rear", "upper", "lower", "left", "right", "transverse", "vertical", "perpendicular", "horizontal", "top", "bottom", etc. indicate orientation or positional relations generally based on the orientation or positional relations as shown in the drawings for ease of description of the present invention and for simplicity of description. In the absence of a statement to the contrary, these directional terms do not indicate and imply that the device or element being referred to must have a particular orientation or be constructed and operated in a particular orientation, and are not to be construed as limiting the scope of protection of the present invention. The directional terms "inner" and "outer" refer to inner and outer relative to the contour of each component itself.

For ease of description, spatially relative terms, such as "on", "above", "on an upper surface of", "upper", etc., may be used herein to describe a spatial relation of a device or feature to other devices or features as illustrated in the figures. It should be understood that the spatially relative terms are intended to include different orientations of the device in use or operation besides the orientation depicted in the figures. For example, under the condition that a device in the figures is inverted, devices described as "above" or "on" other devices or structures would then be oriented "below" or "under" the other devices or structures. Thus, the illustrative terms "above" can include both the orientation of "above" and "below". The device may be otherwise oriented (rotated 90 degrees or at other orientations) and the spatially relative descriptors used herein are interpreted accordingly.

Moreover, it is to be noted that the terms "first", "second", etc. are used to define parts merely for facilitating distinction between the corresponding parts, and the terms do not have a special meaning and are therefore not to be construed as limiting the scope of protection of the present invention if not otherwise stated.

## Claims

1. An amplifying and testing device, comprising:
a support (100), being provided with a test window (110);
a test device (200) arranged on the support (100) and located at the test window (110);
a reagent loading device (300), comprising a bracket (310) and a carrier (320) removably arranged on the bracket (310), the bracket (310) is movably arranged on the support (100) in a transverse direction, and the carrier (320) moves along with the bracket (310), such that the carrier (320) has a storage position located below the test window (110) and a loading position moving out of the support (100); and
a heating device (400) movably arranged on the support (100) in a vertical direction and capable of driving the carrier (320) to lift, so as to lift the carrier (320) to a test position cooperating with the test device (200) in a butting manner, which is contact cooperating or cooperating with a certain gap, wherein
a first shielding member is circumferentially provided on the support (100), the first shielding member (120) shields circumferential outsides of the test window (110) and the reagent loading device (300), the first shielding member (120) is provided with an opening (130) for avoiding the bracket (310) and the carrier (320), such that the bracket (310) can pass through the opening (130) of the first shielding member (120) and move in the transverse direction, the heating device (400) comprises a heating assembly (410), a driving assembly (420) for driving the heating assembly (410) to move, and a second shielding member (430) arranged on a side of the heating assembly (410), and when the driving assembly (420) drives the heating assembly (410) to move to make the carrier (320) located at the test position, the second shielding member (430) shields the opening (130).

2. The amplifying and testing device according to claim 1, wherein the reagent loading device (300) further comprises a transmission mechanism (380), and the bracket (310) is movably arranged on the support (100) in the transverse direction by the transmission mechanism (380).

3. The amplifying and testing device according to claim 2, wherein the transmission mechanism (380) comprises an electric motor (381), a driving wheel (382), a driven wheel (383), and a transmission belt sleeving outsides of the driving wheel (382) and the driven wheel (383), the electric motor (381) is arranged on the support (100), the driving wheel (382) is arranged on an output shaft of the electric motor (381), the driven wheel (383) is arranged on the support (100), and the transmission belt is in driving connection with the bracket (310).

4. The amplifying and testing device according to claim 3, wherein a connection member (340) is arranged on the bracket (310), and the connection member (340) is fixedly connected with the transmission belt.

5. The amplifying and testing device according to claim 4, wherein the transmission belt comprises a synchronous belt (384), and the connection member (340) is provided with a slot (350) cooperating with a tooth of the synchronous belt (384) in an insertion manner.

6. The amplifying and testing device according to claim 1, wherein a guide structure (500) is arranged between the bracket (310) and the support (100).

7. The amplifying and testing device according to claim 6, wherein the guide structure (500) comprises a guide rail (510) and a guide groove (520) cooperating with the guide rail (510), one of the guide rail (510) and the guide groove (520) is arranged on the bracket (310), and the other one of the guide rail (510) and the guide groove (520) is arranged on the support (100).

8. The amplifying and testing device according to claim 1, wherein the bracket (310) is provided with a placing opening (360) corresponding to the test window, the carrier (320) is removably arranged at the placing opening (360), and the placing opening (360) avoids the heating device (400).

9. The amplifying and testing device according to claim 1, wherein the test device (200) comprises a heating cover (210) arranged at the test window (110), and a test assembly (220) arranged on the heating cover (210), and when the carrier (320) is lifted to the test position cooperating with the heating cover (210) in the butting manner, a part of the test assembly (220) is located directly above a reagent hole of the carrier (320).

10. The amplifying and testing device according to claim 1, wherein the support comprises a frame assembly for the heating device (400), the frame assembly comprises:
an outer frame (10), comprising a top plate (11) and a bottom plate (12) that are spaced, and a first support member (13) and a second support member (14) arranged between the top plate (11) and the bottom plate (12), and the top plate (11), the bottom plate (12), the first support member (13) and the second support member (14) define a lifting space;
an inner frame (20), comprising a third support member (21) and a fourth support member (22) spaced between the bottom plate (12) and the top plate (11);
a lifting frame member (30), being liftably located in the lifting space, and the lifting frame member (30) comprises a lifting plate (37) liftably penetrating the third support member (21) and the fourth support member (22), a first support guide member (31) penetrating the third support member (21), and a second support guide member (32) penetrating the fourth support member (22), the first support guide member (31) and the second support guide member (32) are both connected with the lifting plate (37), the third support member (21), the fourth support member (22) and the lifting plate (37) define a placing space; and
a driving assembly (420), being arranged on the bottom plate (12) and being in driving connection with the lifting plate (37).

11. The amplifying and testing device according to claim 10, wherein the driving assembly (420) is a linear motor, and the lifting plate (37) is connected with an output shaft (41) of the linear motor.

12. The amplifying and testing device according to claim 11, wherein the lifting plate (37) is provided with a connection hole (371), and the output shaft (41) is fixedly connected in the connection hole (371).

13. The amplifying and testing device according to claim 12, wherein the connection hole (371) comprises a first circular hole section (372) and a second circular hole section (373) in communication with the first circular hole section (372), and a diameter of the first circular hole section (372) is less than a diameter of the second circular hole section (373), the output shaft (41) is provided with an insert (42), and the insert (42) is capable of moving from the first circular hole section (372) to a position cooperating with the second circular hole section (373) in an insertion manner.

14. A carrier loading method for amplifying and testing, wherein the carrier is loaded by the amplifying and testing device according to any one of claims 1-13, and the carrier loading method for amplifying and testing comprising:
placing the carrier (320) on the bracket (310) of the reagent loading device (300);
moving the bracket (310) in the transverse direction, and stopping moving the bracket (310) when the bracket (310) transfers the carrier (320) to the storage position below the test window (110) of the support (100); and
moving the heating device (400) in the vertical direction, wherein the heating device (400) lifts the carrier (320) at the storage position, and stopping moving the heating device (400) when the heating device (400) lifts the carrier (320) to the test position cooperating with the test device (200) in the butting manner.

## Patentansprüche

1. Verstärkungs- und Testvorrichtung, umfassend:
einen Stützkörper (100), der mit einem Testfenster (110) versehen ist;
eine Testvorrichtung (200), die auf dem Stützkörper (100) angeordnet ist und sich an dem Testfenster (110) befindet;
eine Reagenzladevorrichtung (300), umfassend eine Halterung (310) und einen auf der Halterung (310) lösbar angeordneten Träger (320), wobei die Halterung (310) in Querrichtung bewegbar auf dem Stützkörper (100) angeordnet ist und sich der Träger (320) gemeinsam mit der Halterung (310) derart bewegt, dass der Träger (320) eine Lagerungsposition, die sich unterhalb des Testfensters (110) befindet, und eine Ladeposition, die sich aus dem Stützkörper (100) heraus bewegt, aufweist, und
eine Heizvorrichtung (400), die in vertikaler Richtung bewegbar auf dem Stützkörper (100) angeordnet und fähig ist, den Träger (320) so anzutreiben, dass er angehoben wird, sodass der Träger (320) in eine Testposition angehoben wird, die mit der Testvorrichtung (200) in einander anliegender Weise zusammenwirkt, wobei es sich um eine Kontaktzusammenwirkung oder eine Zusammenwirkung mit einem bestimmten Spalt handelt, wobei
ein erstes Abschirmelement umfangseitig an dem Stützkörper (100) vorgesehen ist, das erste Abschirmelement (120) Außenseiten des Testfensters (110) und der Reagenzladevorrichtung (300) umfangseitig abschirmt, das erste Abschirmelement (120) mit einer Öffnung (130) zum Vermeiden der Halterung (310) und des Trägers (320) derart versehen ist, dass die Halterung (310) die Öffnung (130) des ersten Abschirmelements (120) passieren und sich in Querrichtung bewegen kann, wobei die Heizvorrichtung (400) eine Heizbaugruppe (410), eine Antriebsbaugruppe (420), um die Heizbaugruppe (410) in Bewegung zu versetzen, und ein zweites Abschirmelement (430) umfasst, das an einer Seite der Heizbaugruppe (410) angeordnet ist, und das zweite Abschirmelement (430) die Öffnung (130) abschirmt, wenn die Antriebsbaugruppe (420) die Heizbaugruppe (410) in Bewegung versetzt, um dafür zu sorgen, dass sich der Träger (320) an der Testposition befindet.

2. Verstärkungs- und Testvorrichtung nach Anspruch 1, wobei die Reagenzladevorrichtung (300) ferner einen Übertragungsmechanismus (380) umfasst und die Halterung (310) durch den Übertragungsmechanismus (380) in Querrichtung bewegbar an dem Stützkörper (100) angeordnet ist.

3. Verstärkungs- und Testvorrichtung nach Anspruch 2, wobei der Übertragungsmechanismus (380) einen Elektromotor (381), ein Antriebsrad (382), ein angetriebenes Rad (383) und einen Übertragungsriemen umfasst, der Außenseiten des Antriebsrads (382) und des angetriebenen Rads (383) umschließt, wobei der Elektromotor (381) auf dem Stützkörper (100) angeordnet ist, das Antriebsrad (382) an einer Abtriebswelle des Elektromotors (381) angeordnet ist, das angetriebene Rad (383) an dem Stützkörper (100) angeordnet ist und der Übertragungsriemen in Antriebsverbindung mit der Halterung (310) steht.

4. Verstärkungs- und Testvorrichtung nach Anspruch 3, wobei ein Verbindungselement (340) an der Halterung (310) angeordnet ist und das Verbindungselement (340) fest mit dem Übertragungsriemen verbunden ist.

5. Verstärkungs- und Testvorrichtung nach Anspruch 4, wobei der Übertragungsriemen einen Synchronriemen (384) umfasst und das Verbindungselement (340) mit einem Schlitz (350) versehen ist, der mit einem Zahn des Synchronriemens (384) in einfügender Weise zusammenwirkt.

6. Verstärkungs- und Testvorrichtung nach Anspruch 1, wobei eine Führungsstruktur (500) zwischen der Halterung (310) und dem Stützkörper (100) angeordnet ist.

7. Verstärkungs- und Testvorrichtung nach Anspruch 6, wobei die Führungsstruktur (500) eine Führungsschiene (510) und eine mit der Führungsschiene (510) zusammenwirkende Führungsnut (520) umfasst, wobei entweder die Führungsschiene (510) oder die Führungsnut (520) an der Halterung (310) angeordnet ist und das andere der beiden Elemente - Führungsschiene (510) oder Führungsnut (520) - an dem Stützkörper (100) angeordnet ist.

8. Verstärkungs- und Testvorrichtung nach Anspruch 1, wobei die Halterung (310) mit einer dem Testfenster entsprechenden Platzierungsöffnung (360) versehen ist, der Träger (320) lösbar an der Platzierungsöffnung (360) angeordnet ist und die Platzierungsöffnung (360) die Heizvorrichtung (400) vermeidet.

9. Verstärkungs- und Testvorrichtung nach Anspruch 1, wobei die Testvorrichtung (200) eine an dem Testfenster (110) angeordnete Heizabdeckung (210) und eine an der Heizabdeckung (210) angeordnete Testbaugruppe (220) umfasst und sich ein Teil der Testbaugruppe (220) direkt oberhalb eines Reagenzlochs des Trägers (320) befindet, wenn der Träger (320) in die mit der Heizabdeckung (210) in einander anliegender Weise zusammenwirkende Testposition angehoben ist.

10. Verstärkungs- und Testvorrichtung nach Anspruch 1, wobei der Stützkörper eine Rahmenbaugruppe für die Heizvorrichtung (400) umfasst und die Rahmenbaugruppe Folgendes umfasst:
einen Außenrahmen (10), der eine oberseitige Platte (11) und eine unterseitige Platte (12), die beabstandet sind, und ein erstes Stützelement (13) und ein zweites Stützelement (14), die zwischen der oberseitigen Platte (11) und der unterseitigen Platte (12) angeordnet sind, umfasst und die oberseitige Platte (11), die unterseitige Platte (12), das erste Stützelement (13) und das zweite Stützelement (14) einen Hebebereich definieren;
einen Innenrahmen (20), umfassend ein drittes Stützelement (21) und ein viertes Stützelement (22), die zwischen der unterseitigen Platte (12) und der oberseitigen Platte (11) beabstandet sind;
ein Heberahmenelement (30), das sich hebbar in dem Hebebereich befindet, wobei das Heberahmenelement (30) eine Hebeplatte (37), die das dritte Stützelement (21) und das vierte Stützelement (22) hebbar durchdringt, ein erstes Stützführungselement (31), das das dritte Stützelement (21) durchdringt, und ein zweites Stützführungselement (32), das das vierte Stützelement (22) durchdringt, umfasst, wobei das erste Stützführungselement (31) und das zweite Stützführungselement (32) beide mit der Hebeplatte (37) verbunden sind und das dritte Stützelement (21), das vierte Stützelement (22) und die Hebeplatte (37) einen Platzierungsbereich definieren, und
eine Antriebsbaugruppe (420), die auf der unterseitigen Platte (12) angeordnet und in Antriebsverbindung mit der Hebeplatte (37) ist.

11. Verstärkungs- und Testvorrichtung nach Anspruch 10, wobei die Antriebsbaugruppe (420) ein Linearmotor ist und die Hebeplatte (37) mit einer Abtriebswelle (41) des Linearmotors verbunden ist.

12. Verstärkungs- und Testvorrichtung nach Anspruch 11, wobei die Hebeplatte (37) mit einem Verbindungsloch (371) versehen ist und die Abtriebswelle (41) fest in dem Verbindungsloch (371) verbunden ist.

13. Verstärkungs- und Testvorrichtung nach Anspruch 12, wobei das Verbindungsloch (371) einen ersten kreisförmigen Lochabschnitt (372) und einen zweiten kreisförmigen Lochabschnitt (373) umfasst, der mit dem ersten kreisförmigen Lochabschnitt (372) in Kommunikation steht, und ein Durchmesser des ersten kreisförmigen Lochabschnitts (372) kleiner ist als der Durchmesser des zweiten kreisförmigen Lochabschnitts (373), die Abtriebswelle (41) mit einem Einsatz (42) versehen ist und der Einsatz (42) fähig ist, sich von dem ersten kreisförmigen Lochabschnitt (372) in eine Position zu bewegen, die mit dem zweiten kreisförmigen Lochabschnitt (373) in einfügender Weise zusammenwirkt.

14. Trägerladeverfahren für Verstärken und Testen, wobei der Träger durch die Verstärkungs- und Testvorrichtung nach einem der Ansprüche 1-13 geladen wird und das Trägerladeverfahren für Verstärken und Testen Folgendes umfasst:
Platzieren des Trägers (320) auf der Halterung (310) der Reagenzladevorrichtung (300);
Bewegen der Halterung (310) in Querrichtung und Anhalten der Bewegung der Halterung (310), wenn die Halterung (310) den Träger (320) in die Lagerposition unterhalb des Testfensters (110) des Stützkörpers (100) überführt, und
Bewegen der Heizvorrichtung (400) in vertikaler Richtung, wobei die Heizvorrichtung (400) den Träger (320) an der Lagerposition anhebt, und Anhalten der Bewegung der Heizvorrichtung (400), wenn die Heizvorrichtung (400) den Träger (320) in die mit der Testvorrichtung (200) in einander anliegender Weise zusammenwirkende Testposition anhebt.

## Revendications

1. Dispositif d'amplification et d'essai, comprenant :
un support (100), muni d'une fenêtre de test (110) ;
un dispositif de test (200) disposé sur le support (100) et situé au niveau de la fenêtre de test (110) ;
un dispositif de chargement de réactifs (300), comprenant un élément de fixation (310) et un élément porteur (320) monté de manière amovible sur l'élément de fixation (310), l'élément de fixation (310) étant monté de manière mobile sur le support (100) dans une direction transversale, et l'élément porteur (320) se déplaçant conjointement avec l'élément de fixation (310), de telle sorte que l'élément porteur (320) présente une position de stockage située sous la fenêtre de test (110) et une position de chargement en se délaçant hors du support (100) ; et
un dispositif de chauffage (400) monté de manière mobile sur le support (100) dans une direction verticale et capable d'entraîner l'élément porteur (320) vers le haut, de manière à amener l'élément porteur (320) dans une position de test en coopération avec le dispositif de test (200) d'une manière par aboutement, qui est en coopération par contact ou en coopération avec un certain écart, dans lequel
un premier élément de protection est prévu de manière circonférentielle sur le support (100), le premier élément de protection (120) protégeant les bords extérieurs de la fenêtre de test (110) et du dispositif de chargement de réactifs (300), le premier élément de protection (120) est muni d'une ouverture (130) destinée à éviter l'élément de fixation (310) et l'élément porteur (320), de telle sorte que l'élément de fixation (310) puisse passer à travers l'ouverture (130) du premier élément de protection (120) et se déplacer dans la direction transversale, le dispositif de chauffage (400) comprend un ensemble de chauffage (410), un ensemble d'entraînement (420) destiné à entraîner l'ensemble de chauffage (410) pour se déplacer, et un deuxième élément de protection (430) disposé sur un côté de l'ensemble de chauffage (410), et lorsque l'ensemble d'entraînement (420) entraîne l'ensemble de chauffage (410) pour se déplacer afin de placer l'élément porteur (320) en position de test, le deuxième élément de protection (430) protège l'ouverture (130).

2. Dispositif d'amplification et d'essai selon la revendication 1, dans lequel le dispositif de chargement de réactifs (300) comprend en outre un mécanisme de transmission (380), et l'élément de fixation (310) est agencé de manière mobile sur le support (100) dans la direction transversale par l'intermédiaire du mécanisme de transmission (380).

3. Dispositif d'amplification et d'essai selon la revendication 2, dans lequel le mécanisme de transmission (380) comprend un moteur électrique (381), une roue motrice (382), une roue menée (383) et une courroie de transmission s'enroulant autour des faces extérieures de la roue motrice (382) et de la roue menée (383), le moteur électrique (381) est monté sur le support (100), la roue motrice (382) est montée sur un arbre de sortie du moteur électrique (381), la roue menée (383) est montée sur le support (100), et la courroie de transmission est en liaison d'entraînement avec l'élément de fixation (310).

4. Dispositif d'amplification et d'essai selon la revendication 3, dans lequel un élément de liaison (340) est disposé sur l'élément de fixation (310), et l'élément de liaison (340) est relié de manière fixe à la courroie de transmission.

5. Dispositif d'amplification et d'essai selon la revendication 4, dans lequel la courroie de transmission comprend une courroie synchrone (384), et l'élément de liaison (340) est muni d'une fente (350) coopérant par insertion avec une dent de la courroie synchrone (384).

6. Dispositif d'amplification et d'essai selon la revendication 1, dans lequel une structure de guidage (500) est disposée entre l'élément de fixation (310) et le support (100).

7. Dispositif d'amplification et d'essai selon la revendication 6, dans lequel la structure de guidage (500) comprend un rail de guidage (510) et une rainure de guidage (520) coopérant avec le rail de guidage (510), l'un parmi le rail de guidage (510) et la rainure de guidage (520) est disposé sur l'élément de fixation (310), et l'autre parmi le rail de guidage (510) et la rainure de guidage (520) est disposé sur le support (100).

8. Dispositif d'amplification et d'essai selon la revendication 1, dans lequel l'élément de fixation (310) est muni d'une ouverture de placement (360) correspondant à la fenêtre de test, l'élément porteur (320) est disposé de manière amovible au niveau de l'ouverture de placement (360), et l'ouverture de placement (360) évite le dispositif de chauffage (400).

9. Dispositif d'amplification et d'essai selon la revendication 1, dans lequel le dispositif de test (200) comprend un couvercle chauffant (210) disposé au niveau de la fenêtre de test (110), et un ensemble de test (220) disposé sur le couvercle chauffant (210), et lorsque l'élément porteur (320) est soulevé jusqu'à la position de test en coopérant avec le couvercle chauffant (210) d'une manière par aboutement, une partie de l'ensemble de test (220) est située directement au-dessus d'un trou à réactif de l'élément porteur (320).

10. Dispositif d'amplification et d'essai selon la revendication 1, dans lequel le support comprend un ensemble de châssis pour le dispositif de chauffage (400), l'ensemble de châssis comprend :
un châssis extérieur (10), comprenant une plaque supérieure (11) et une plaque inférieure (12) qui sont espacées l'une de l'autre, ainsi qu'un premier élément de support (13) et un deuxième élément de support (14) disposés entre la plaque supérieure (11) et la plaque inférieure (12), et la plaque supérieure (11), la plaque inférieure (12), le premier élément de support (13) et le deuxième élément de support (14) définissent un espace de levage ;
un châssis intérieur (20), comprenant un troisième élément de support (21) et un quatrième élément de support (22) espacés entre la plaque inférieure (12) et la plaque supérieure (11) ;
un élément de châssis de levage (30), situé de manière à pouvoir être soulevé dans l'espace de levage, et l'élément de châssis de levage (30) comprend une plaque de levage (37) pénétrant de manière à pouvoir être soulevée dans le troisième élément de support (21) et le quatrième élément de support (22), un premier élément de guidage de support (31) pénétrant dans le troisième élément de support (21), et un deuxième élément de guidage de support (32) pénétrant dans le quatrième élément de support (22), le premier élément de guidage de support (31) et le deuxième élément de guidage de support (32) étant tous deux reliés à la plaque de levage (37), le troisième élément de support (21), le quatrième élément de support (22) et la plaque de levage (37) définissant un espace de placement ; et
un ensemble d'entraînement (420), étant disposé sur la plaque inférieure (12) et étant en liaison d'entraînement avec la plaque de levage (37).

11. Dispositif d'amplification et d'essai selon la revendication 10, dans lequel l'ensemble d'entraînement (420) est un moteur linéaire, et la plaque de levage (37) est reliée à un arbre de sortie (41) du moteur linéaire.

12. Dispositif d'amplification et d'essai selon la revendication 11, dans lequel la plaque de levage (37) est pourvue d'un trou de liaison (371), et l'arbre de sortie (41) est relié de manière fixe dans le trou de liaison (371).

13. Dispositif d'amplification et d'essai selon la revendication 12, dans lequel le trou de liaison (371) comprend une première section de trou circulaire (372) et une deuxième section de trou circulaire (373) en communication avec la première section de trou circulaire (372), et le diamètre de la première section de trou circulaire (372) est inférieur au diamètre de la deuxième section de trou circulaire (373), l'arbre de sortie (41) est muni d'un insert (42), et l'insert (42) est apte à se déplacer depuis la première section de trou circulaire (372) vers une position coopérant avec la deuxième section de trou circulaire (373) par insertion.

14. Procédé de chargement d'élément porteur pour l'amplification et l'essai, dans lequel l'élément porteur est chargé par le dispositif d'amplification et d'essai selon l'une quelconque des revendications 1 à 13, et le procédé de chargement d'élément porteur pour l'amplification et l'essai comprenant :
le placement de l'élément porteur (320) sur l'élément de fixation (310) du dispositif de chargement de réactifs (300) ;
la déplacement de l'élément de fixation (310) dans la direction transversale, et l'arrêt du déplacement de l'élément de fixation (310) lorsque l'élément de fixation (310) transfère l'élément porteur (320) vers la position de stockage située sous la fenêtre de test (110) du support (100) ; et
le déplacement du dispositif de chauffage (400) dans la direction verticale, dans lequel le dispositif de chauffage (400) soulève le support (320) à la position de stockage, et l'arrêt du déplacement du dispositif de chauffage (400) lorsque le dispositif de chauffage (400) soulève l'élément porteur (320) jusqu'à la position de test, en coopérant avec le dispositif de test (200) de la manière par aboutement.
